# EUROPEAN PATENT APPLICATION

(11) **EP 4 661 025 A1**
(43) Date of publication of application: **10.12.2025**
(21) Application number: 24179524.4
(22) Date of filing: 03.06.2024
(51) Int. Cl.: G16H 50/20, G16H 50/30, G16H 50/50, G16B 40/30

(54) **COMPUTER-IMPLEMENTED METHOD FOR THE ESTIMATION OF A RISK HEART RHYTHM DISORDER IN A PATIENT'S HEART**

(71) Applicant: Inheart, 33600 Pessac (FR)
(72) Inventor: CEDINILK, Nicolas, 33600 PESSAC (FR)
(74) Representative: Aquinov

(57) **Abstract**

The invention concerns a computer-implemented method for the estimation of a risk of heart rhythm disorder in a patient's heart, the method comprising: (S03) receiving a mapping of points (IH) representing a tissue of said heart and each being labelled with a value (Tᵢ) and/or a classification (Cᵢ) indicating a local characteristic; (S2) simulating the propagation of electric signals from inducing locations (ILⱼ), to which is applied virtual induction protocol (IPₖ) ; (S3) detecting from each simulation outcome (EAM_{j,k}) whether a self-sustained arrhythmia is induced; (S5) a step of clustering, from simulation outcomes (EAM_{j,k}), inducible sites into groups (Gₗ) of similar inducible sites; (S6) a step of computing, for each group (Gₗ) of similar inducible sites (ILⱼ) and from the number (N) of inducible sites of said group, a risk value (RVₗ) indicating whether a heart rhythm disorder can occur. Figure to be published with the abstract: Figure 1

## Description

The present invention relates to the field of computer assisted medical risk stratification. More particularly, the invention relates to a computer-implemented method for the estimation of a risk of heart rhythm disorder in a patient's heart.

A malfunction of the cardiac conduction system can lead to abnormal heart rhythms or cardiac arrhythmias, such as ventricular tachycardia (VT), or atrial fibrillation (AF). The scar from a cardiomyopathy, for instance from a previous heart attack, might form abnormal electrical circuits within the heart, which causes VT. Such arrythmias might cause sudden cardiac death, also named SCD. Several treatments, drug-based or involving surgery, might be considered, depending on the seriousness of the arrythmia.

For instance, it is possible to treat arrhythmias with an invasive catheter-based ablation. This ablation consists of inserting a catheter into the heart through veins or arteries to burn an area of the heart tissue that is causing arrhythmias. This procedure requires an assessment of the cardiac region contributing to the arrhythmia and subsequently cauterizing a region of cardiac muscle to disrupt the arrhythmia. Even if this procedure is commonly used, it carries risks due to its invasive nature, its complexity, and its length.

For patients presenting a risk for SCD, an implantable cardioverter-defibrillator, also named ICD, can be used to prevent arrythmias. However, identification of patients admissible to ICD is crucial, since the placement of an ICD involves many risks, as surgical complications, and inappropriate shocks.

Inducibility of arrhythmias in clinical electrophysiology study (EPS) has been used for heart rhythm disorder diagnosis and risk stratification, to help cardiologists to determine the appropriate treatment which minimizes the risks for the patient. Although this method presents many benefits, its applicability is limited by its invasiveness and its cost. Noninvasive methods can also help the cardiologist, as an electrocardiogram (ECG) analysis to evaluate the heart rhythm or a cardiac image analysis to evaluate the left-ventricular ejection fraction (LFEV). But these methods require strong analysis skills and lack precision, especially for early detection and prevention.

There is therefore a need for a non-invasive method to correctly detect or predict a heart rhythm disorder and to stratify the risk of this heart rhythm disorder, even at an early stage of the arrythmia.

The object of the present invention is to answer to this need.

For this purpose, the subject of the invention is a computer-implemented method for the estimation of a risk of heart rhythm disorder in a patient's heart, the method comprising:
a. a step of receiving at least one mapping of points representing a tissue of said patient's heart, each point being labelled with at least a value of at least one parameter and/or a classification indicating a local characteristic of said tissue at or around said point position;
b. a step of simulating the propagation of electric signals in said mapping of points from each of a plurality of inducing locations within said mapping of points, to which is applied at least one virtual induction protocol, wherein each simulation outcome is associated to a couple of inducing location and induction protocol;
c. detecting from each simulation outcome whether a self-sustained arrhythmia is induced from the application of the associated induction protocol to the associated inducing location and classifying said associated inducing location as an inducible site whether a self-sustained arrythmia is detected in said associated simulation outcome;
d. a step of clustering, from the simulation outcomes associated to the inducible sites, said inducible sites into groups of similar inducible sites which induce similar simulations outcomes according to a given similarity metric;
e. a step of computing, for each group of similar inducible sites, a risk value associated to said group indicating whether a heart rhythm disorder can occur, said risk value being computed at least from the number of inducible sites of said group.

According to the invention, a mapping of points of a tissue of a patient's heart is provided, for instance after being computed from a 3D image of the patient's heart. This 3D representation is associated to local features which are representative, directly, or indirectly and partly, or fully, of features of the tissue which can lead to the presence or the absence of arrythmias. These local features might be physiological, geometrical, electrical and/or structural characteristic, resulting directly from the points intensities and/or contrast and/or from computing on the mapping of points, and/or from another data acquisition technique.

An example of local characteristic might be the thickness of the tissue, for instance determined with measuring the distance separating closest points of inner and outer surface of a wall of the tissue. In the case of a wall of the heart, as the myocardium, the thickness of the wall may help to identify areas responsible of arrythmias, such as scar, chamber's wall segment particularly thin or morphological isthmuses, i.e., areas having a thickness lower than the thickness of adjacent zones. In particular, such isthmuses might create electrical pathway problems on the heart conduction system, such as re-entry circuits.

An example of classification might be the cellular composition of the tissue. For example, in the case of some cardiac diseases, part of the muscular tissue is replaced by adipocytes or by calcified structures or by fibrosis; such structural changes tend to weaken the thickness of the heart as well and might be responsible of arrhythmias.

This mapping of points can be then used to assess arrhythmia inducibility of the patient in silico, by conducting virtual electrical stimulations at different inducing locations with one or more induction protocols. From each simulation, the propagation of the wave can be observed to check whether a virtual arrhythmia, namely a re-entrant wave, is virtually induced from an inducible site. Each simulation can therefore virtually induce an arrhythmia, helping to stratify the risk of heart rhythm disorder in a noninvasive, economical and risk-free way.

To this end, inducible sites leading to similar arrythmias are grouped, considering that the number of inducible sites which generate a similar arrythmia gives an indication about the likelihood of a heart rhythm disorder caused by this arrythmia. The invention then takes advantage from the fact that a large number of inducible sites within a same group indicates a more likely heart rhythm disorder than a low number.

A risk value, in the form of a score or an index, can then be inferred from each group or cluster of inducible sites, from characteristics extracted from this group and at least the number of inducible sites contained in this group. All risk values can then be combined to compute a global risk value for the patient or to generate an augmented 3D model of the patient's heart in which the risk values are added.

In the context of the present specification, unless expressly provided otherwise, a "computer" may refer, but is not limited to, a desktop computer, a laptop computer, a tablet computer, a piece of testing equipment, a network appliance, a controller, a digital signal processor, a computational engine within an appliance, a consumer-electronic device, a portable computing device, and/or another electronic device, and/or any combination thereof appropriate to the relevant task at hand. Moreover, the method steps might be all executed on a same device. As a variant, the method steps might be executed on several devices, connected by wires or by a wireless connection.

In the context of the present specification, unless expressly provided otherwise, a "mapping of points representing a tissue" is a set of points digitally representing this tissue, said points can be encoded computationally by sequences or lists of numerical values, in particular sequences of at least one numerical value representing the coordinates of said points in a particular reference. The coordinates can be specified in any type of chart, for instance in cartesian, spherical cylindrical coordinates or any other type of geometrical chart in one, two or three spatial dimensions. A mapping of points might be a 2D mapping of points or a 3D mapping of points.

A 3D mapping of points might be a "heart 3D model", namely one or more structured mesh, each comprising a plurality of vertices connected with each other to define a plurality of faces and/or volumes which approximate internal and/or external surfaces and/or volumes of all or part of the heart. A 3D model might be a triangle mesh or a quadrilateral mesh, or more generally a polygonal mesh, which comprises a plurality of vertices, with edges connecting pairs of vertices and/or polygonal faces connecting a closed set of vertices. As a variant, a 3D model might be a tetrahedral mesh or a hexahedral mesh or a prism mesh, or more generally a volume mesh, which comprises a plurality of vertices, with polyhedrons connecting a closed set of polygons defined by closed set of vertices. Preferably, each vertex and/or edge and/or polygon and/or polyhedron might be labelled as being a part of a specific anatomical part of the heart. A 3D model might be stored as a list of vertices each associated with spatial coordinates and with a set of connections to other vertices, or as a list of vertices associated with spatial coordinates and a list of polygons or faces each defined by a subset of vertices contained in said list of vertices, being understood that any other suitable method of storage might be used in the context of the present specification.

A 3D mapping of points might be an unstructured set of points, namely a point cloud, where each point might be or not connected to another point of the point cloud.

In the context of the present specification, said "3D mapping of points" might be (or might be computed from) one of, or a combination of two or more of : an electrophysiological map, resulting from an electrocardiogram (ECG) and/or from an invasive mapping, an anatomical and/or a functional map, resulting from a computed tomography(CT)-scan, a spectral computed tomography(SCT)-scan, a positron emission tomography(PET)-scan, a single photon emission computed tomography(SPECT)-scan, a photon-counting computed tomography(PCCT)-scan or a magnetic resonance imaging(MRI)-scan, and an electro-anatomical map, resulting from an invasive mapping.

In the context of the present specification, unless expressly provided otherwise, "a parameter indicating a local characteristic of a tissue at a point position or around a point position" might be an electrical, a physiological, a physical or a geometrical feature of an area of the tissue whose location corresponds to said position. In the context of a heart tissue as the myocardium, an electrical feature might be an activation time, an electric potential, or a conduction velocity. A physiological feature might be a tissue density. A physical or a geometrical feature might be a tissue thickness, such as a myocardial thickness. It is understood that any other suitable local feature of the heart, which is representative, directly, or indirectly and partly, or fully, of the presence or the absence of arrythmias, might be used in the context of the present specification.

According to one embodiment, said parameter value associated to a point of said mapping of points indicates a local thickness of said tissue at or around said point position.

In the context of the present specification, unless expressly provided otherwise, a "classification indicating a local characteristic of a tissue at a point position or around a point position" might be a class indicating the type of cells composing the tissue at or around a location corresponding said position and/or a class indicating the major type of cells composing the tissue at or around a location corresponding said position and/or a class indicating the proportion of the types of cells composing the tissue at or around a location corresponding said position. In the context of a heart tissue as the myocardium, said type of tissue might be a muscle tissue, a fat tissue, a fibrose tissue, or a calcified tissue. As a variant, a "classification indicating a local characteristic of a tissue at a point position or around a point position" might be a label previously attributed to a point and indicated whether a part of the tissue at or around said point position belongs to a cardiomyopathy scar or a myocardial fibrosis.

According to one embodiment, said classification associated to a point of said mapping of points indicates the presence of fat, calcification, fibrosis and/or muscle in said tissue at or around said point position.

According to one embodiment, the method comprises a preliminary step of acquiring a 3D image and/or recording a 3D image of a patient's heart or a region of his heart, the mesh being computed from said 3D image. The 3D image may be acquired directly from an image acquisition device such as a CT-scan or MRI. Alternatively, the 3D image may be obtained from a recording medium on which it is stored, such as a local memory or a distant database, the 3D image having been acquired beforehand the method.

For example, the step of acquiring the 3D image can be performed by tomography. These techniques are also identified as CT-scan, SCT-scan, PCCT-scan, PET-scan, SPECT-scan, or CAT-scan and are based on the measurement of X-ray absorption by the tissues of an organ. Tomography provides a plurality of 2D images each representing a slice of the organ, which are then combined to reconstruct the 3D image of the anatomical structure of the observed organ. The 3D image comprises a volumetric distribution of pixels, or voxels. Two or more 3D images might be acquired from distinct tomography techniques, or modalities, to build then a multimodal and/or a multidimensional 3D image.

The method according to the invention can thus comprise a 3D modeling step of the 3D image of the heart to generate a 3D model forming then the mapping of points. The 3D modelling step comprises a modeling step of at least one layer, or one wall of the heart shown in the 3D image, such as an inner face and an outer face of the myocardium of the heart. Said 3D model might be a mesh, especially a polygonal or a polyhedron mesh, of said layer, especially of said inner and outer faces.

According to the invention, a thickness value at a point position or around a point position might be determined with computing the distance between the inner face and the outer face at said position. A classification of the type of tissue at a point position or around a point position might be determined with considering the Hounsfield units encoding the radiodensity at said position.

In the context of the present specification, unless expressly provided otherwise, "the application of virtual induction protocol at an inducing location of a mapping of points" may refer, but is not limited to, the computing of one or more electrophysiological feature of the patient's heart, for instance for each point of said mapping of points, which depends on the propagation of a virtual electrical signal, for instance a virtual depolarization wave, induced from the application of one or more virtual electrical stimulation at said inducing location, throughout the mapping of points. Said propagation might be computed considering a given cardiac electrophysiology model configured or combined with the mapping of points. Said electrophysiological features might be an activation map of the heart and/or the variation over time of the transmembrane potential across the heart and/or an electrocardiogram of the heart.

A virtual induction protocol might be a virtual train of regular electrical pulses followed by one or more extra pulses whose interval to the last regular pulse is controlled. Said protocol might be, for instance, a part of a S1S2 protocol or a S1S2S3 protocol.

In a S1S2 protocol, in an initial controlled pacing phase (called S1), few stimulations are applied to the inducing location, after which an earlier stimulation (called S2) is applied. The first phase S1 is repeated and the interval to the second phase S2 is reduced at each iteration, until either an arrhythmia is induced, or the interval reaches the refractory period. In a S1S2S3 protocol, the interval to the S2 stimulus is set slightly above the refractory period, and a third stimulus (called S3) is applied in the same way the S2 was applied in the S1S2 protocol.

According to one embodiment, the method comprises a sampling step of said mapping of points with a regular grid, said inducing locations being determined from the sampled points.

Advantageously, the sampling step comprises, for each grid's compartment, the selection of one of the points of the mapping of points which belong to said grid's compartment, based on the parameter's values and/or the classifications associated to these points, wherein each selected point forms an inducing location. For instance, the sampling step comprises, for each grid's compartment, the selection of the point of the mapping points which belongs to said grid's compartment and which presents the lowest parameter's values. This sampling method maximizes the chance of an inducing location to induce an arrhythmia.

According to one embodiment, said simulation step comprises, for each simulation associated to a couple of inducing location and induction protocol, the computing of the variation over time of an electrical potential at each point of said mapping of points, wherein the electrical potential's variation over time at each point of said mapping of points is computed from a cardiac cell electrical potential model and from an electrical potential propagation model, at least the cardiac cell electrical potential model being parameterized with said parameter value and/or said classification associated to said point.

According to this embodiment, each simulation outcome might comprise a map of the electrical potential's variation over time at each point of said mapping of points. Alternatively, each simulation outcome might comprise any local and/or global appropriate representation of the cardiac electrical activity which can be computed from said electrical potential's variation over time at each point of said mapping of points, as an activation map, a reentry circuit and/or an electrocardiogram. As non-limitative example, an electrocardiogram might be computed with placing a pair of virtual electrodes at appropriate points of said mapping of points and with subtracting the signal acquired by one of the electrodes, from the variation of electrical potential of each simulation outcome, to the other.

For instance, said electrical potential variation at each point might be iteratively computed from a starting time at which said virtual induction protocol is applied at said inducing location and until a stop condition is reached, based on said cardiac cell electrical potential model, from the previous electrical potential at said point and from the previous electrical potentials at the neighboring points of said point, said neighboring electrical potentials being propagated to said point according to said electrical potential propagation model. Said stop condition might the detection of a self-sustained arrythmia, the reach of a predefined number of iterations and/or the reach of a predefined interval of the induction protocol, as the refractory period.

According to a non-limitative example, the cardiac cell electrical potential model might be a « ionic model », as a Mitchell-Schaeffer model of the cardiac action potential, which describes the ion fluxes tending to depolarize or repolarize the cell's membrane. Said cardiac cell electrical potential model, when used for the computing of the electrical potential variation at a point, might be parameterized depending on the parameter value and/or said classification associated to said point.

For instance, said model might comprise a differential equation representing the variation over time of the transmembrane potential from:
a. ionic fluxes causing the depolarization of the cardiomyocyte membrane, said fluxes depending on an excitability parameter which mimic the excitability threshold of cardiomyocytes;
b. ionic fluxes causing the repolarization of the cardiomyocyte membrane.

Said differential equation might be the following: $\frac{dv}{dt} = {J}_{in} \left(v, h\right) + {J}_{out} \left(v\right) + {J}_{stim}$ ${J}_{in} \left(v, h\right) = \frac{h \left(v\left(v-λ\right)\left(1-v\right)\right)}{{τ}_{in}}$ ${J}_{out} \left(v\right) = - \frac{v}{{τ}_{out}}$ wherein v represents the scaled transmembrane potential, Jᵢₙ represents the ionic movements that tend to depolarize the cardiomyocyte membrane, Jₒᵤₜ represents the ionic movements that tend to repolarize the cardiomyocyte membrane, Jₛₜᵢₘ represents the external stimulation current induction protocol, h is a gating variable controlling the recovery of the virtual cardiac cell, τᵢₙ represents the depolarization current speed, λ is the excitability parameter which controls the excitability of virtual cardiomyocytes and τₒᵤₜ represents the repolarization current speed.

One or more of the parameters h, τᵢₙ, λ and τₒᵤₜ might be computed for each point of the mapping of points, depending on the parameter value and/or the classification associated to said point.

For instance, for the computing of the electrical potential variation at a point of the mapping of points, the parameter τᵢₙ might be computed following an inverse relationship with the parameter value associated to said point. When said parameter value is the myocardium thickness, the cardiac cell electrical potential model will then increase the propagation speed of the depolarization wave at thick areas and a low and decrease the propagation speed of the depolarization wave at thin areas.

The value of the parameter τₒᵤₜ might be computed from the value of the parameter τᵢₙ to keep constant the ratio between these parameters.

For the computing of the electrical potential variation at a point of the mapping of points, the parameter λ might be computed depending on the parameter value and/or the classification associated to said point. For instance, said parameter λ might have lower values for points associated to a classification indicating healthy tissue, as "muscle", or to high myocardium thickness values, than values for points associated to a classification indicating in-farcted tissue, as "fibrosis", or to low myocardium thickness values.

Parameter h acts as a gating variable to controls the recovery of the virtual cardiac cell, by modulating its excitability and general response to an external current. Said parameter value might be computed from the following equation: $\frac{dh}{dt} = { \begin{matrix}\frac{1 - v}{{τ}_{open}} if v < {v}_{gate} \\ \frac{v}{{τ}_{close}} if v > {v}_{gate}\end{matrix}$ wherein τₒₚₑₙ and τ_{close} represent respectively the membrane opening and closing timings, and v_{gate} is the minimum current to be applied to the membrane to initiate the depolarization of the cell.

Said parameters τₒₚₑₙ, τ_{close} and v_{gate} might be set as predefined constant or computed from the other parameters values.

Further cardiac cell electrical potential models, as a Luo-Rudy model, might be considered without going beyond the scope of the present invention.

According to a non-limitative example, the electrical potential propagation model might be based on a Lattice Boltzmann Method. Said method is based on an recursive iteration of a collision step, in which the electrical potential at each point of said mapping of points is computed from a cardiac cell electrical potential model and from the previous computed electrical potential at said point, and on a streaming step, in which a predefined part of the electrical potential at each point is diffused to the point's neighbors according to predefined diffusion directions to compute new electrical potential at each point.

For instance, the electrical potential at each point might be diffused either in an isotropic way or in an anisotropic way to the point's neighbors according to six diffusion directions corresponding to a cuboid.

Further electrical potential propagation models, as an Eikonal model, might be considered without going beyond the scope of the present invention.

According to one embodiment, in said detection step, a self-sustained arrhythmia is detected from a simulation outcome whether a value indicating a cardiac electrical activity, computed from the electrical potential at each point of said mapping of points of said simulation, is greater than a predefined threshold for longer than a predefined duration.

As a non-limitative example, said detection step might be implemented simultaneously with the simulation step, for instance at the end of each iterative step of the simulation step. As an example, a self-sustained arrhythmia might be detected whether the sum and/or the average of the electrical potentials computed for the whole mapping of points stays above than a predefined threshold for longer than a predefined duration, as 2 seconds.

As a variant, said detection step might be implemented following the simulation step, from an analysis of an activation map contained in the simulation outcome or of one or more electrocardiograms computed from the electrical potential variation at each point.

According to one embodiment, said simulation step comprises the simulation of the propagation of electric signals in said mapping of points for each of a plurality of inducing locations within said mapping of points and for each of a plurality of virtual induction protocols, wherein each simulation outcome is associated to a couple of inducing location and induction protocol. Said plurality of virtual induction protocols might form a S1S2 protocol or a S1S2S3 protocol. In the context of the invention, further virtual induction protocols might be considered, as S1S2S3S4 protocol or any appropriate succession of virtual induction protocols.

As an example, each induction protocol of virtual induction protocols might be sequentially applied to a same inducing location to achieve a set of simulation outcomes for said inducing location. Each induction protocol might consist in a virtual train of regular electrical pulses followed by one or more extra pulses whose interval to the last regular pulse is controlled so this is reduced from one induction protocol to the following.

Advantageously, the simulations of the propagation of electric signals in said mapping of points for an inducing location are run sequentially for each of said plurality of virtual induction protocols until a self-sustained arrhythmia is detected from a simulation outcome.

Advantageously, for the implementation of the simulation based on said plurality of virtual induction protocols, part or whole of the simulation outcome based on the application of the regular pulses is kept in memory from one simulation to the other. Said feature implements a memoization which helps to speed up the computation, since the simulation is computed only for the extra pulse.

According to one embodiment, the simulation comprises a step of computing, from each of the simulation outcomes associated to the inducible sites, a graphical representation of a cardiac electrical activity associated to said inducible site. In said clustering step, inducible sites with similar associated graphical representation of a cardiac electrical activity according to a given similarity metric are clustered into a same group of similar inducible sites. According to these features, simulation outcomes and inducible sites might be compared and clustered based on a similarity of the cardiac electrical activity.

According to a non-limitative example, said graphical representation of a cardiac electrical activity is an electrocardiogram and said metric is based on the correlation between distinct electrocardiograms. 12-lead electrocardiograms can be easily computed from a simulation outcome and offer a comparable representation of a cardiac electrical activity.

Advantageously, said clustering step might comprise: the computing, from each simulation outcome associated with an inducible site, of an electrocardiogram; the computing of a cycle length of each electrocardiogram; the computing of a circular correlation coefficient between each pair of electrocardiograms based on the cycle length of each electrocardiogram; the clustering of the inducible sites into groups of similar inducible sites based on the circular correlation coefficients.

A circular correlation coefficient between two signals might be computed with evaluating values of a cross-correlation function between one signal and a shifted version of the other signal, said values of the cross-correlation function being evaluated with a plurality of values of the shift variable.

For instance, each electrocardiogram might be computed with placing a pair of virtual electrodes at appropriate points of said mapping of points and with subtracting the signal acquired by one of the electrodes, from the variation of electrical potential of each simulation outcome, to the other.

For instance, said cycle length of an electrocardiogram might computed from the evaluation of the maximum of an auto-correlation function of said electrocardiogram.

For instance, two inducible sites might be clustered in a same group whether the circular correlation coefficient of their associated electrocardiograms is above a predefined threshold.

As a variant, the inducible sites might be clustered with implementing a hierarchical clustering algorithm, wherein the distance metric is based on said circular correlation coefficient.

Further metrics to compare electrocardiograms, as a Pearson correlation coefficients, might be considered without going beyond the scope of the present invention. Further graphical representations with an appropriate metric for comparison, as an activation map, might be considered without going beyond the scope of the present invention. The similarity between two representations might be estimated by any suitable method, including machine learning algorithms appropriately trained to compare graphical representations.

According to one embodiment, said risk value associated to each group is computed at least from the number of inducible sites of said group and from an inducible capability of each inducible site of said group. This inducible capability is a weight which represents the capacity of an inducible site to cause a self-sustained arrythmia.

Said inducible capability might be computed based on the induction protocol which has caused the self-sustained arrhythmia in the simulation outcome associated to said inducible site, for instance with being selected on the among a plurality of values each associated to an induction protocol.

According to one embodiment, the method comprises a step of computing, from the simulation outcomes associated to at least one of the inducible sites of each group, a re-entry circuit associated to said group. Said risk value associated to each group is computed at least from the number of inducible sites of said group and from a likelihood factor computed from the re-entry circuit associated to said group and which indicates the likelihood of a heart rhythm disorder caused by said re-entry circuit.

Said re-entry circuit might be computed from the variation over time of the electrical potential at each point of said mapping of points to identify a group of points of said mapping of points defining a closed circuit in which an electrical signal circulates continuously during the simulation.

Said likelihood factor might be computed, for instance, from the length of the re-entry circuit, determined for example from the number of points forming said re-entry circuit, and/or from the location of the re-entry circuit in the mapping of points, for instance from its proximity to predefined anatomical parts of the heart.

According to an example, the method comprises a step of computing, from the risk values associated to each group indicating whether a heart rhythm disorder can occur, a global risk value indicating whether said patient's heart presents a heart rhythm disorder. Said global risk value might be provided to a cardiologist to help him to diagnose or predict a heart rhythm disorder.

Said global risk value might be computed, for instance, from the sum of the risk values associated to the groups.

According to another example, the method comprises a step of computing, from the simulation outcomes associated to at least one of the inducible sites of each group, a re-entry circuit associated to said group, and a step of adding to said mapping of points each re-entry circuit, said re-entry circuit being labelled with the risk values associated to the group associated to said re-entry circuit.

Said combination of mapping of points, re-entry circuits and risk values form then a new 3D model of the patient's heart, that can be appropriately used by a cardiologist to diagnose or predict a heart rhythm disorder and/or to prepare a cardiac intervention, for instance to identify catheter ablation targets.

The subject of the invention is also a computing device for the implementation of the method according to the invention, the computing device comprising a memory arranged to receive at least one mapping of points representing a tissue of said patient's heart; and a computing unit arranged to implement at least the simulation step, the detection step, the clustering step, and the risk value computing step of said method.
Figure 1 is a logic flow diagram that depicts a computer-implemented method, according to an embodiment of the invention;
Figure 2 shows a schematic representation of a 3D mapping of points representing a tissue of a patient's heart;
Figure 3 shows a schematic representation of different inducing locations in the 3D mapping of point of Figure 2;
Figure 4 shows a schematic representation of a virtual induction protocol;
Figure 5 shows a schematic representation of a cardiac cell electrical potential model;
Figure 6 shows a schematic representation an electrical potential propagation model;
Figure 7 shows a schematic representation of a part of a simulation outcome resulting from the application of the induction protocol of Figure 6 to an inducing location of Figure 3;
Figure 8 shows a schematic representation of simulated electrocardiograms computed from different simulation outcomes; and

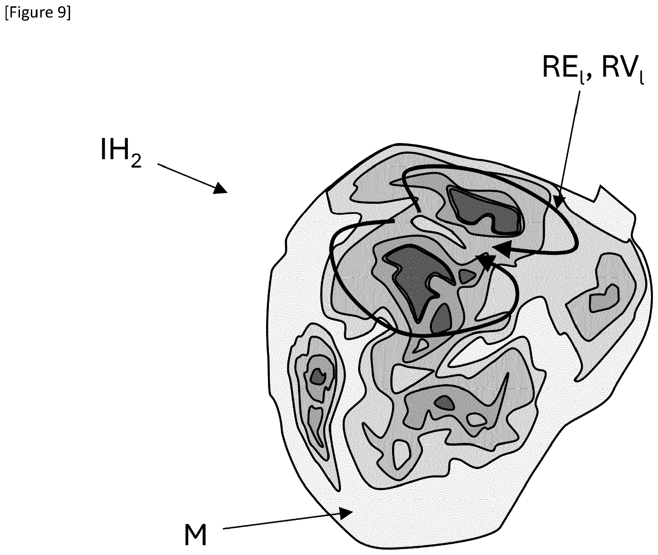

Figure 9 shows a schematic representation of a re-entry circuit superimposed on the 3D mapping of points of Figure 2.

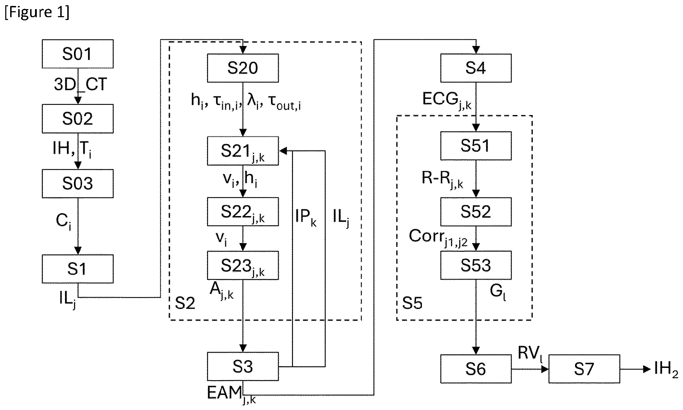

Reference is made to Figure 1 which shows a computer-implemented method for the generation of a combined representation of a 3D image of an organ and a 3D anatomical model of said organ. In the depicted example, the organ is a heart, although the method can be implemented for any kind of organ with no or slight modification.

In a preliminary step S01, a first 3D image 3D_CT of the patient's heart has been acquired from a CT-scan method. Said method provides a plurality of 2D images each representing a slice of the heart, which are then combined to reconstruct the 3D image of the heart.

Any suitable imaging method can be used to replace the CT-scan method, as a SCT-scan method, a PET-scan method, a SPECT-scan method, a PCCT-scan method, a MRI-scan method or an electro-anatomical study acquired during an intracardiac catheter intervention.

In a second preliminary step S02, a 3D mapping of point IH of the patient's heart is generated from the first 3D image 3D_CT. In the depicted embodiment, said 3D mapping of point is a 3D model of the heart IH, in which each vertex is associated to local features which are representative, directly, or indirectly and partly, or fully, of features of the organ, such as the presence or the absence of arrythmias in case of a heart. These local features might be physiological, geometrical, electrical and/or structural characteristic, resulting directly from computing on the mapping of points and/or from another data acquisition technique.

More precisely, an inner surface or layer and an outer surface or layer of the patient's heart myocardium have been modelized from the 3D image 3D_CT with defining a plurality of vertices of meshes representing said surfaces. This modeling results in a mesh M of the inner surface or layer or of the outer surface or layer of the myocardium, wherein each vertex Pᵢ of the mesh M is therefore labelled with the value of the thickness Tᵢ of the myocardium, meaning the thickness computed from these inner and outer surfaces or layers, at the location of the heart corresponding to the position of this vertex.

In the depicted example, these vertices have been segmented, according to their thickness. Said mesh M comprises then a plurality of superimposed sub-meshes SMᵢ, with each sub-mesh corresponding to a thickness range of the myocardium.

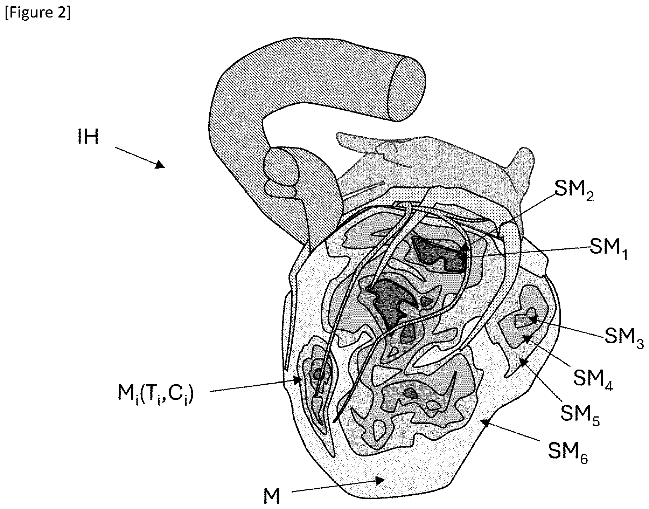

Reference is made to Figure 2 which shows an example of a 3D model IH. The model IH comprises a mesh M comprising six superimposed thickness sub-meshes SM₁ to SM₆ corresponding each to a range of myocardium thickness, namely 5mm to 6mm, 4mm to 5mm, 3mm to 4mm, 2mm to 3mm, 1mm to 2mm, and 0 to 1mm. For description conciseness, regions of greater thickness have been depicted darker than regions of lower thickness.

Moreover, in a further preliminary step S03, each point of the first 3D image 3D_CT has been associated to a numerical value in terms of Hounsfield units encoding the radiodensity at said point. Since calcification type cells, fat type cells and muscle type cells do not present a same radiodensity, it is therefore possible to discriminate, for a slice of the myocardium at a defined location of the heart, the proportion of calcification, fat and muscle type cells composing said slice. Each vertex Pᵢ of the mesh M has been therefore labelled with a class Cᵢ indicating the major type of cells composing the myocardium at the location of the heart corresponding to the position of this vertex.

In the depicted example, said class Cᵢ is selected among classes C₁ "calcification tissue", C₂ "fat tissue", C₃ "fibrosis tissue", C₄ "muscle tissue".

While thickness Tᵢ of the myocardium and cells type classes Cᵢ define, for a vertex Pᵢ of the mesh M, two local characteristics of the heart at a location corresponding to said vertex Pᵢ, the invention is not limited to these local characteristics and only one or more than two local characteristics can be used to label a vertex Pᵢ.

For instance, a vertex Pᵢ can be labelled with a value of a local electrical feature, an activation time, an electric potential, or a conduction velocity, as value of a physiological feature, as a tissue density; or with a classification indicating whether the myocardium at vertex location has a cardiomyopathy scar or a myocardial fibrosis; or any other suitable local feature which might be used by a physician in the context of the present specification.

One can note that the steps S01 to S02 might be executed by another computing unit than the one used for the rest of the method. Moreover, steps S01 to S02 might be executed fully automatically by a computing unit or with a human assistance. The resulting 3D model IH might be stored on a recording medium, such as a memory of the computer or an external memory support or a distant database through which it is accessible to the computer.

At the end of the step S03, the 3D model IH is provided to the computing unit of the computer.

In a sampling step S1, a plurality of inducing locations ILⱼ are selected among the vertices Pᵢ of the mesh M.

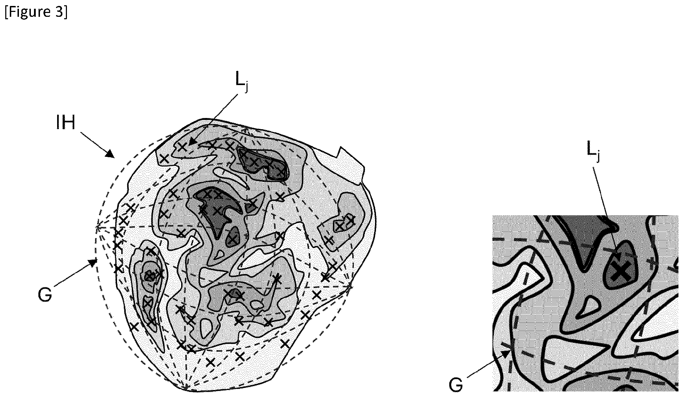

Reference is made to Figure 3 which shows an example of selection of inducing locations ILⱼ.

In said sampling step S1, a grid G is positioned on the mesh M with matching its apex with the left ventricular apex. For each grid's compartment, the vertex Pᵢ which belongs to said grid's compartment and which presents the lowest thickness Tᵢ is selected as an inducing location ILⱼ.

The right part of Figure 3 shows a zoomed view of the mesh M provided with the grid G, wherein the vertex Pᵢ with the lowest thickness Tᵢ has been selected.

In a step S2, the computing unit implements simulations of the propagation of electric signals, namely a depolarization wave front, in the 3D model IH, following the application of virtual induction protocols IPₖ, namely trains of electrical pulses, at each of the inducing locations ILⱼ.

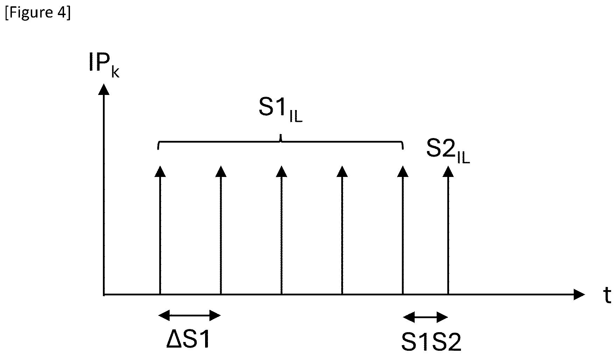

For each simulation, a virtual induction protocol IPₖ is applied to an inducing location ILⱼ. Reference is made to Figure 4 which shows an example of a virtual induction protocol IPₖ.

In an initial controlled pacing phase (called S1_{IL}), a train of regular electrical pulses is applied to the inducing location ILⱼ, each regular pulse being separated from the previous with an interval ΔS1 for instance of 600 ms. After the initial phase, an extra pulse (called S2 _{IL}) is applied to the inducing location ILⱼ, whose interval S1S2 to the last regular pulse S1 is controlled to be lower than the regular interval.

For the next simulation, following the application of this virtual induction protocol IPₖ, a new virtual induction protocol IPₖ₊₁ is applied at the same inducing location ILⱼ. In this new virtual induction protocol, the first phase S1_{IL} is identically repeated and the interval to the second phase S2_{IL} is reduced.

All the induction protocols applied to a same inducing location ILⱼ forms thus a S1S2 protocol, in which each protocol is sequentially applied, the interval of the extra pulse S2_{IL} to the last regular pulse S1_{IL} being reduced from one induction protocol to the following. The simulation step S2 comprises thus two loops of simulation wherein all the protocols IPₖ of the whole S1S2 protocol are sequentially applied to each inducing location ILⱼ.

For each simulation, the computing unit determines the variation over time of the action potential, or the transmembrane potential, at each vertex Pᵢ, from a cardiac cell electrical potential model and from an electrical potential propagation model.

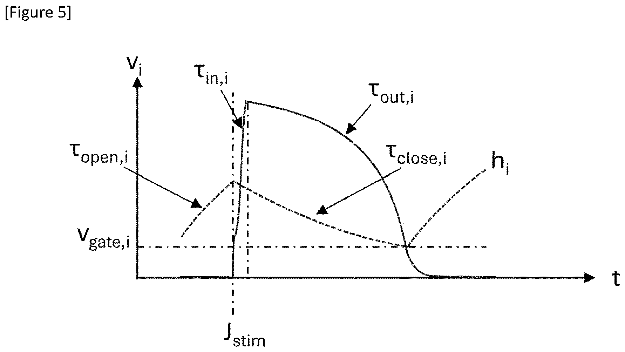

Reference is made to Figure 5, which represents an example of cardiac cell electrical potential model, namely a Mitchell-Schaeffer model of the cardiac action potential, in which the variation over time of the transmembrane potential of a cell corresponding to a vertex Pᵢ is described with the following: $\frac{{dv}_{i}}{dt} = {J}_{in} \left({v}_{i}, {h}_{i}\right) + {J}_{out} \left({v}_{i}\right) + {J}_{stim}$ ${J}_{in} \left({v}_{i}, {h}_{i}\right) = \frac{{h}_{i} \left({v}_{i}\left({v}_{i}-{λ}_{i}\right)\left(1-{v}_{i}\right)\right)}{{τ}_{in , i}}$ ${J}_{out} \left({v}_{i}\right) = - \frac{{v}_{i}}{{τ}_{out , i}}$
vᵢ represents the scaled transmembrane potential of a virtual cardiac cell corresponding to a vertex Pᵢ, Jᵢₙ represents the ionic movements that tend to depolarize the cardiomyocyte membrane, Jₒᵤₜ represents the ionic movements that tend to repolarize the cardiomyocyte membrane, Jₛₜᵢₘ represents an external stimulation current of an induction protocol, hᵢ is a gating variable controlling the recovery of the virtual cardiac cell, τ_{in,i} represents the depolarization current speed, λᵢ is the excitability parameter which mimic the excitability threshold of cardiomyocytes and τ_{out,i} represents the repolarization current speed.

Figure 5 depicts the variation over time of the transmembrane potential vᵢ and of the gating variable hᵢ, following the application of an electrical pulse Jₛₜᵢₘ.

It can be seen than the parameters hᵢ, τ_{in,i}, λᵢ and τ_{out,i} control the steepness of the slope of v, during the depolarization phase (Pho) and the minimal value needed to initiate the depolarization, and therefore the way a depolarization wave front propagates through the cell.

Since there is a relationship between the type of tissue and the thickness of the heart wall and the propagation of the depolarization wave front, the cardiac cell electrical potential model can be parameterized to reflect this relationship.

Therefore, in a first sub-step S20, the parameters hᵢ, τ_{in,i}, λᵢ and τ_{out,i} are computed for each vertex Pᵢ, depending on its associated thickness Tᵢ and classification Cᵢ. This set-up sub-step S20 is implemented once for all the simulations.

As example, the parameter τ_{in,i} might be computed following an inverse relationship with the thickness Tᵢ, the parameter τ_{out,i} might be set as 10 times the value of the parameter τᵢₙ, the parameter λᵢ might be computed to have lower values for vertices Tᵢ associated to a classification C₄ than vertices Tᵢ associated to a classification C₁, C₂ or C₃.

Parameter hᵢ acts as a gating variable to controls the recovery of the virtual cardiac cell, by modulating its excitability and general response to an external current, and is computed from the following equation: $\frac{{dh}_{i}}{dt} = { \begin{matrix}\frac{1 - {v}_{i}}{{τ}_{open , i}} if {v}_{i} < {v}_{gate , i} \\ \frac{{v}_{i}}{{τ}_{close , i}} if {v}_{i} > {v}_{gate , i}\end{matrix}$ wherein τ_{open,i} and τ_{close,i} represent respectively the membrane opening and closing timings, and v_{gate,i} is the minimum current to be applied to the membrane to initiate the depolarization of the cell.

Said parameters τ_{open,i}, τ_{close,i} and v_{gate,i} might be set during the set-up sub-step S20 as predefined constant.

To propagate the depolarization wave front, the simulation is implemented with an electrical potential propagation model.

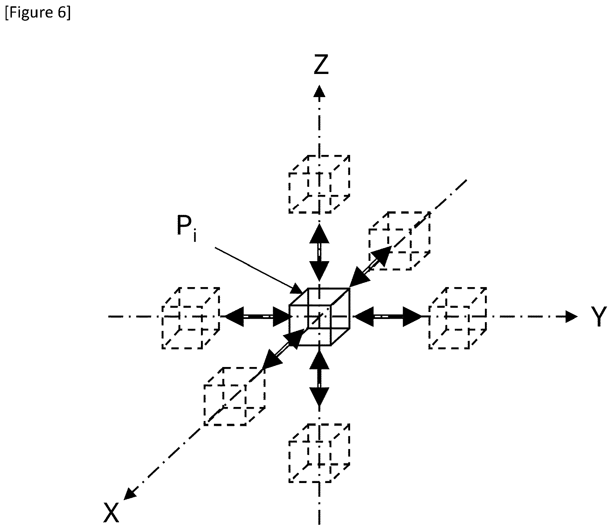

Reference is made to Figure 6, which represents an example of electrical potential propagation model in which, in a streaming step, a predefined part of the electrical potential at each vertex Pᵢ is diffused to its neighbors while a part stays at said vertex Pᵢ.

In the example depicted in Figure 6, an equal part of the electrical potential, namely an eight, at each vertex Pᵢ is diffused to the point's neighbors according to six diffusion directions X, Y, Z, while a part, namely a quarter, stays at the vertex Pᵢ.

The electrical potential vᵢ at each vertex Pᵢ can be then iteratively computed from a starting time at which a virtual induction protocol IPₖ is applied at an inducing location ILⱼ.

At each iteration, in a sub-step S21_{j,k}, the variation of the electrical potential vᵢ from its previous value is computed for each vertex Pᵢ, from the cardiac cell electrical potential model as shown in Figure 4. In the case where the iteration corresponds to the timing of the application of an electrical pulse to the inducing location ILⱼ, the component Jₛₜᵢₘ of the cardiac cell electrical potential model is updated accordingly.

During the subs-step S21_{j,k}, the gating variable hᵢ is also updated.

In a sub-step S22_{j,k}, the electrical potential of each vertex Pᵢ, computed at sub-step S21_{j,k}, is diffused to its neighbors and the new value electrical potential vᵢ of each vertex Pᵢ is determined with summing the portion staying at said vertex Pᵢ and the portion diffused from the neighbors toward said vertex Pᵢ.

The iterations of these sub-steps S21_{j,k} and S22_{j,k} are continued until a stop condition is reached. Said stop condition can be either of the detection of a self-sustained arrythmia or the reach of a predetermined number of iterations or of a predetermined duration of the simulation without any cardiac electrical activity detected.

In a sub-step S23_{j,k}, implemented at each iteration from the application of the last electrical pulse of the induction protocol IPₖ, it is checked whether an electrical activation persists in the 3D model. A value A_{j,k} is determined as the sum of the electrical potentials vᵢ computed at the end of the sub-step S22_{j,k}, for all vertices Pᵢ.

This value A_{j,k} is compared to a threshold and a self-sustained arrhythmia is detected in a step S3 whether the value A_{j,k} stays above the threshold for a number of iterations or a simulation duration longer than 2 seconds. The detection of a self-sustained arrhythmia stops both the current simulation and the loop of simulations related to a same inducing locations ILⱼ and starts a new loop of simulations with applying the induction protocols IPₖ to the next inducing location ILⱼ₊₁. On the contrary, the reach of a predetermined number of iterations or of a predetermined duration of the simulation without any cardiac electrical activity being detected stops the simulation and starts the next simulation of the current loop with applying the next induction protocols IPₖ₊₁ to the same inducing location ILⱼ.

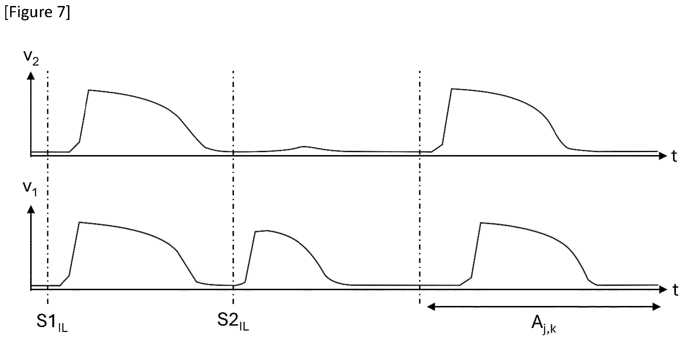

Reference is made to Figure 7 which depicts the variation of the electrical potentials vᵢ computed for two vertices Pᵢ, the lower graph being related to a vertex P₁ closer to the inducing location ILⱼ than the vertex P₂ related to the upper graph. As it can be seen, the application of the extra pulse S2_{IL} results in a self-sustaining electrical activity, due to the propagation of the depolarization wave front in a re-entry circuit in which said vertex P₁ and P₂ are located.

In the depicted embodiment, the detection step S3 is thus implemented simultaneously with the simulation step S2. As undepicted variants, the simulation step S3 might be implemented after the completion of the whole simulation step S2, the complete series of induction protocols IPₖ might be applied to a same inducing locations ILⱼ even if a self-sustained arrhythmia is detected for one of these induction protocols IPₖ.

Within a loop of simulations related to a same inducing locations ILⱼ, the variation of the electrical potentials vᵢ due to the initial controlled pacing phase S1_{IL} is computed once for all vertices Pᵢ. This variation is kept in memory from one simulation of this loop to the other and only the variation of the electrical potentials vᵢ due to the second pulse S2_{IL} is computed at each simulation.

When the S1S2 interval of an induction protocols IPₖ reaches a refractory period, the loop of simulations related to a same inducing locations ILⱼ is terminated and a new loop of simulations related to the next inducing locations ILⱼ₊₁ begins.

At the end of the detection step S3, whether a self-sustained arrhythmia is detected, the variation over time of the electrical potential vᵢ at each vertex Pᵢ of the 3D model IH is assembled in an activation map EAM_{j,k} associated to the inducing location ILⱼ, now being an inducible site ILⱼ, and the induction protocol IPₖ which have both induced the arrythmia.

In a step S4, different graphical representations of a cardiac electrical activity associated to each inducible site ILⱼ are computed from the associated activation map EAM_{j,k}.

More precisely, the computing unit evaluates a 12-lead electrocardiogram ECG_{j,k} from each activation map EAM_{j,k}.

The 12-lead electrocardiogram ECG_{j,k} might be computed with placing virtual electrodes at appropriate vertex Pᵢ and with subtracting, for each couple of electrodes, the signals acquired by one electrode, estimated from the variation of electrical potential vᵢ of the corresponding vertex Pᵢ, to the other.

As undescribed variant, the computing unit might derive from the activation map EAM_{j,k} any local and/or global appropriate representation of the cardiac electrical activity.

In a step S5, the activation maps EAM_{j,k} and their associated inducible sites ILⱼ are clustered into groups Gₗ of similar inducible sites, based on the similarities of the corresponding electrocardiogram ECG_{j,k}.

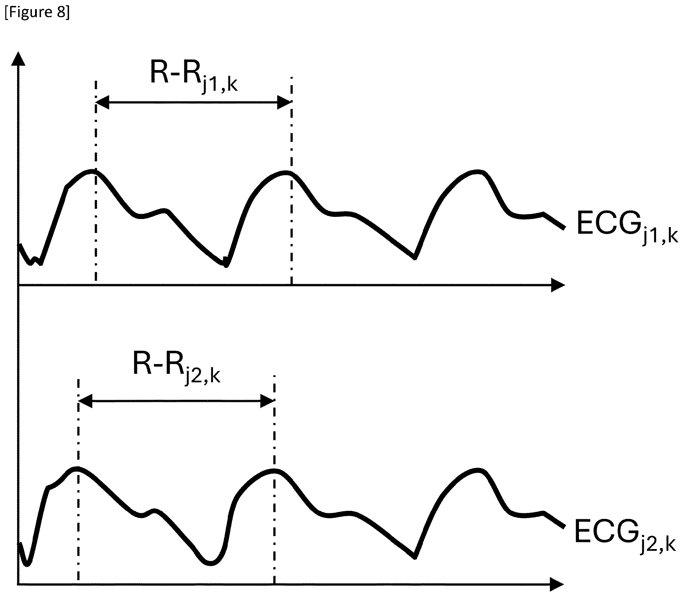

Reference is made to Figure 8 which depicts aVL electrocardiograms extracted from two 12-lead electrocardiogram ECG_{j1,k} and ECG_{j2,k} derived from two distinct activation maps EAM_{j1,k} and EAM_{j2,k}.

In a first sub-step S51, a cycle length R-R_{j,k} is extracted from each electrocardiogram ECG_{j,k}.

Each cycle length R-R_{j,k} is computed from the evaluation of the maximum of an auto-correlation function of the electrocardiogram ECG_{j,k}.

In a second sub-step S52, for each couple of electrocardiograms ECG_{j1,k} and ECG_{j2,k}, the computing unit evaluates a shifted circular correlation coefficient Corr_{j1,j2} between these electrocardiograms ECG_{j1,k} and ECG_{j2,k}, from the maximum of a shifted correlation function of ECG_{j1,k} and ECG_{j2,k} computed on the previously estimated cycle length R-R_{j1,k} or R-R_{j2,k}.

In a third sub-step S53, inducible sites ILⱼ are clustered into groups Gₗ of similar inducible sites based on these circular correlation coefficients Corr_{j1,j2}.

In the depicted example, the clustering sub-step S53 is implemented with a hierarchical clustering algorithm, wherein the distance metric is defined by 1 - ∥*Corr*_{*j*1*,j*2}∥ and wherein the cluster separation threshold has been set to 0,05.

In other words, at the end of the clustering sub-step S53, each group Gₗ contains all the inducible sites ILⱼ whose associated electrocardiograms ECG_{j,k} have a two-wise distance metric lower than 5%.

In a step S6, a risk value RVₗ is computed for each group Gₗ of similar inducible sites ILⱼ.

Said risk value RVₗ is computed from the number N of inducible sites ILⱼ within the group Gₗ, an inducible capability wⱼ of each these inducible sites ILⱼ and from a likelihood factor Lₗ of the group Gₗ.

The inducible capability is a weight which represents the capacity of an inducible site to cause a self-sustained arrythmia, considering that a self-sustained arrythmia induced from an induction protocol with a high S1S2 interval reflects a more inducible arrythmia than self-sustained arrythmia induced from an induction protocol with a low S1S2 interval. Thus, the inducible capability wⱼ of an inducible site ILⱼ is computed based on the induction protocol IPₖ which has caused the self-sustained arrhythmia in the associated simulation outcome EAM_{j,k}, for instance with being selected on the among a plurality of values each associated to an induction protocol EAM_{j,k}.

The likelihood factor is a global weight which indicates the chances for a depolarization wave front to propagate within a re-entry circuit, considering that a long re-entry circuit offers less chances than a short re-entry circuit. Thus, the likelihood factor Lₗ is computed from a length of a re-entry circuit REₗ associated to the group Gₗ. This re-entry circuit REₗ is formed by a group of vertices Pᵢ defining a closed circuit in the 3D model IH in which an electrical signal circulates continuously during a simulation.

The risk value RVₗ can then be computed for instance with the following equation: *RVₗ* = ${L}_{l} . {\sum }_{j = 1}^{N} {w}_{j}$.

In a step S7, each re-entry circuit REₗ and the risk value RVₗ of the associated group Gₗ are added to the 3D model IH, for instance with being superimposed to the mesh M, to form an augmented 3D model IH₂ than can be displayed to a cardiologist to diagnose or predict a heart rhythm disorder and/or to prepare a cardiac intervention, for instance to identify catheter ablation targets.

The methods disclosed herein may also be implemented by software programs executable by a computer system. Further, implementations may include distributed processing and parallel processing, especially for processing in parallel several or all data in the data sets.

The illustrations described herein are intended to provide a general understanding of the structure of various embodiments. These illustrations are not intended to serve as a complete description of all the elements and features of apparatus, processors and systems that utilizes the structures or methods described therein. Many other embodiments or combinations thereof may be apparent to those of ordinary skills in the art upon reviewing the disclosure by combining the disclosed embodiments. Other embodiments may be utilized and derived from the disclosure, such that structural and logical substitutions and changes may be made without departing from the scope of the disclosure.

Further, the disclosure and the illustrations are to be considered as illustrative rather than restrictive, and the appended claims are intended to cover all such modifications, enhancements, and other embodiments, which fall within the true spirit and scope of the description. Thus, the scope of the following claims is to be determined by the broadest permissible interpretation of the claims and their equivalents and shall not be restricted or limited by the foregoing description.

## Claims

1. A computer-implemented method for the estimation of a risk of heart rhythm disorder in a patient's heart, the method comprising:
a. (S03) a step of receiving at least one mapping of points (IH) representing a tissue of said patient's heart, each point (Pᵢ) being labelled with at least a value (Tᵢ) of at least one parameter and/or a classification (Cᵢ) indicating a local characteristic of said tissue at or around said point position;
b. (S2) a step of simulating the propagation of electric signals in said mapping of points from each of a plurality of inducing locations (ILⱼ) within said mapping of points, to which is applied at least one virtual induction protocol (IPₖ), wherein each simulation outcome (EAM_{j,k}) is associated to a couple of inducing location and induction protocol;
c. (S3) detecting from each simulation outcome (EAM_{j,k}) whether a self-sustained arrhythmia is induced from the application of the associated induction protocol (IPₖ) to the associated inducing location (ILⱼ) and classifying said associated inducing location as an inducible site whether a self-sustained arrythmia is detected in said associated simulation outcome;
d. (S5) a step of clustering, from the simulation outcomes (EAM_{j,k}) associated to the inducible sites (ILⱼ), said inducible sites into groups (Gₗ) of similar inducible sites which induce similar simulations outcomes according to a given similarity metric;
e. (S6) a step of computing, for each group (Gₗ) of similar inducible sites (ILⱼ), a risk value (RVₗ) associated to said group indicating whether a heart rhythm disorder can occur, said risk value being computed at least from the number (N) of inducible sites of said group.

2. The method according to claim 1, wherein said parameter value (Tᵢ) associated to a point (Pᵢ) of said mapping of points (IH) indicates a local thickness of said tissue at or around said point position.

3. The method according to one of claims 1 to 2, wherein said classification (Cᵢ) associated to a point (Pᵢ) of said mapping of points (IH) indicates the presence of fat, calcification, fibrosis and/or muscle in said tissue at or around said point position.

4. The method according to one of claims 1 to 3, characterized it comprises a sampling step (S1) of said mapping of points (IH) with a regular grid (G), said inducing locations (ILⱼ) being determined from the sampled points.

5. The method according to one of claims 1 to 4, wherein said simulation step (S2) comprises, for each simulation associated to a couple of inducing location (ILⱼ) and induction protocol (IPₖ), the computing of the variation over time of an electrical potential (vᵢ) at each point (Pᵢ) of said mapping of points (IH), wherein the electrical potential's variation over time at each point (Pᵢ) of said mapping of points (IH) is computed from a cardiac cell electrical potential model and from an electrical potential propagation model, at least the cardiac cell electrical potential model being parameterized with said parameter value (Tᵢ) and/or said classification (Cᵢ) associated to said point.

6. The method according to the previous claim, wherein, in said detection step (S3), a self-sustained arrhythmia is detected from a simulation outcome (EAM_{j,k}) whether a value (A_{j,k}) indicating a cardiac electrical activity, computed from the electrical potential (vᵢ) at each point (Pᵢ) of said mapping of points (IH) of said simulation, is lower than a predefined threshold for longer than a predefined duration.

7. The method according to one of claims 1 to 6, wherein said simulation step (S2) comprises the simulation of the propagation of electric signals in said mapping of points (IH) for each of a plurality of inducing locations (ILⱼ) within said mapping of points and for each of a plurality of virtual induction protocols (IPₖ), wherein each simulation outcome is associated to a couple of inducing location (ILⱼ) and induction protocol (IPₖ).

8. The method according to the previous claim, wherein the simulations of the propagation of electric signals in said mapping of points (IH) for an inducing location (ILⱼ) are run sequentially for each of said plurality of virtual induction protocols (IPₖ) until a self-sustained arrhythmia is detected from a simulation outcome (EAM_{j,k}).

9. The method according to one of claims 1 to 8, characterised it comprises a step (S4) of computing, from each of the simulation outcomes (EAM_{j,k}) associated to the inducible sites (ILⱼ), a graphical representation (ECG_{j,k}) of a cardiac electrical activity associated to said inducible site, and wherein, in said clustering step (S5), inducible sites (ILⱼ) with similar associated graphical representation (ECG_{j,k}) of a cardiac electrical activity according to a given similarity metric are clustered into a same group (Gₗ) of similar inducible sites.

10. The method according to the previous claim, wherein said graphical representation (ECG_{j,k}) of a cardiac electrical activity is an electrocardiogram and wherein said metric is based on the correlation between distinct electrocardiograms.

11. The method according to one of claims 1 to 10, wherein said risk value (RVₗ) associated to each group (Gₗ) is computed at least from the number (N) of inducible sites (ILⱼ) of said group and from an inducible capability (wⱼ) of each inducible site of said group.

12. The method according to one of claims 1 to 11, wherein characterised it comprises a step of computing, from the simulation outcomes (EAM_{j,k}) associated to at least one of the inducible sites (ILⱼ) of each group (Gₗ), a re-entry circuit (REₗ) associated to said group, and wherein said risk value (RVₗ) associated to each group (Gₗ) is computed at least from the number (N) of inducible sites of said group and from a likelihood factor (Lₗ) computed from the re-entry circuit associated to said group and which indicates the likelihood of a heart rhythm disorder caused by said re-entry circuit.

13. The method according to one of claims 1 to 12, characterised it comprises a step of computing, from the risk values (RVₗ) associated to each group (Gₗ) indicating whether a heart rhythm disorder can occur, a global risk value indicating whether said patient's heart presents a heart rhythm disorder.

14. The method according to one of claims 1 to 13, characterised it comprises a step of computing, from the simulation outcomes (EAM_{j,k}) associated to at least one of the inducible sites (ILⱼ) of each group (Gₗ),, a re-entry circuit (REₗ) associated to said group, and a step (S7) of adding to said mapping of points (IH) each re-entry circuit, said re-entry circuit being labelled with the risk values (RVₗ) associated to the group associated to said re-entry circuit.

15. A computing device for the implementation of the method according to one of claims 1 to 14, comprising a memory arranged to receive at least one mapping of points representing a tissue of said patient's heart; and a computing unit arranged to implement at least the simulation step (S2), the detection step (S3), the clustering step (S5), and the risk value computing step (S6).
